# EUROPÄISCHE PATENTSCHRIFT

(11) **EP 1 504 790 B1**
(45) Veröffentlichungstag und Bekanntmachung des Hinweises auf die Patenterteilung: **12.09.2007**
(21) Anmeldenummer: 03016697.9
(22) Anmeldetag: 04.08.2003
(51) Int. Cl.: A61N 2/02, A61B 5/055, A61B 5/0484

(54) **Vorrichtung zum Berechnen einer Energiemenge zur Stimulation des Gehirns**
Apparatus for calculating an energy amount for brain stimulation
Appareil pour calculer une quantité d'énergie pour la stimulation du cerveau

(43) Veröffentlichungstag der Anmeldung: 09.02.2005
(73) Patentinhaber: BrainLAB AG, 85551 Kirchheim/Heimstetten (DE)
(72) Erfinder: Tanner, Philipp, 80796 München (DE); Bauch, Thomas, 80469 Munchen (DE)
(74) Vertreter: Schwabe - Sandmair - Marx

(56) Entgegenhaltungen:
- EP-A- 1 269 913
- EP-A- 1 273 320
- WO-A-02/073526
- US-A1- 2003 050 527

## Beschreibung

Die Erfindung bezieht sich auf eine Vorrichtung zum Berechnen einer Energiemenge für eine Stimulation des Gehirns, insbesondere für eine transcraniale magnetische Stimulation (TMS) eines bestimmten Bereichs des Gehirns.

In verschiedenen medizinischen Bereichen, wie zum Beispiel der Neurologie, der Psychiatrie oder der Gehirn-Chirurgie ist es wünschenswert bestimmte funktionale Bereiche des Gehirns lokalisieren zu können, um beispielsweise Hirnfunktionen zu kartieren. Soll zum Beispiel durch einen chirurgischen Eingriff ein Hirntumor entfernt werden, so sollte der Tumor soweit wie möglich entfernt werden, wobei vor allem sogenannte primäre Hirnareale, welche bezüglich der Motorik, Sensorik, der Sprache oder den visuellen Fähigkeiten einer Person eine entscheidende Rolle spielen, nach Möglichkeit nicht verletzt werden sollen. Diese Bereiche sollten bei einem chirurgischen Eingriff wenn möglich gar nicht oder nur in äußerst geringem Umfang verletzt werden.

Das Auffinden solcher besonderen Hirnbereiche wurde nach einem bekannten direkten Verfahren intra-operativ durchgeführt, wobei mittels Elektroden eine direkte kortikale Stimulation (DCS) an einem geöffneten Schädel erfolgte. Dabei wurde eine Elektrode in einen bestimmten Bereich des Gehirns eingebracht und ein elektrischer Impuls angelegt, wobei die auf den elektrischen Impuls folgende Reaktion der untersuchten Person, zum Beispiel das Zucken eines Muskels oder das Wahrnehmen von visuellen Eindrücken, beobachtet wurde. Die durch die direkte kortikale Stimulation aufgefundenen besonderen Hirnbereiche wurden mittels aufgelegter kleiner Plättchen markiert, welche für einen Chirurgen bei einer nachfolgenden Gehirnoperation eine Orientierungshilfe bezüglich der möglichst nicht zu verletzenden Hirnbereiche darstellten. Die direkte kortikale Stimulation stellt bis zum heutigen Tag das präziseste Verfahren zur Kartierung von Hirnfunktionen dar und ermöglicht eine Genauigkeit bei der Auffindung bestimmter Hirnareale im Bereich von wenigen Millimetern. Jedoch kann dieses Verfahren nur intra-operativ durchgeführt werden, wobei die untersuchte Person bei vollem Bewusstsein sein muss. Dies kann bei der Anwendung dieses Verfahrens jedoch zu Problemen führen, da dieser Zustand für die untersuchte Person unangenehm ist und die Person, falls es zu Komplikationen kommen sollte, aufgrund der geöffneten Schädeldecke nicht einfach hingelegt und ruhig gestellt werden kann.

Es sind weiterhin verschiedene indirekte Verfahren zur Kartierung von Hirnfunktionen bekannt, mit welchen jedoch nur eine erheblich geringere Genauigkeit beim Auffinden spezifischer Hirnareale erreicht werden kann. So muss zum Beispiel bei der funktionellen Kernspintomographie (fMRI) eine untersuchte Person bestimmte Aktionen wie zum Beispiel eine Handbewegung ausführen, welche die Durchblutung der diesen Aktionen zugeordneten Hirnbereichen fördert. Diese Veränderung der Durchblutung bestimmter Hirnbereiche kann aufgrund der Entkopplung von Durchblutung und Sauerstoffverbrauch während der neuronalen Aktivität gemessen werden, da hierdurch eine Hyperoxygenierung und damit ein Abfall der Konzentration des paramagnetischen Deoxy-Hämoglobins (BOLD-Effekt) auftritt, was dann als sogenanntes "endogenes Kontrastmittel" mittels geeigneter Sequenzen mit Kernspintomographie gemessen werden kann. Jedoch ist dieses Verfahren wie oben erwähnt, relativ ungenau und liefert nur eine räumliche Auflösung im Bereich von etwa 0,5 bis 1,0 cm.

Aus Neurosurgery 1992-1998, December 1997, Volume 41, Number 6, 1319 "Stereotactic Transcranial Magnetic Stimulation: Correlation with Direct Electrical Cortical Stimulation" ist ein Verfahren bekannt, bei welchem eine stereotaktische transcraniale magnetische Stimulation (TMS) für das präoperative funktionale Mapping des motorischen Cortex verwendet wird. Der Kopf eines Patienten wird dabei fest und unbeweglich mit einer Kopflehne verbunden, wobei eine Schwenkarm vorgesehen ist, an welchem eine 8-förmige Spule so angebracht ist, dass die Armspitze unter dem Spulenschnittpunkt liegt. Der Arm wird dabei so ausgerichtet, dass die unter dem Schnittpunkt der beiden Spulen liegenden Spitze auf einen bestimmten Bereich zeigt, in welchem ein Strom induziert werden soll.
Aus der US 5,738,625 ist ein Verfahren bekannt, um Nervenzellen magnetisch zu stimulieren.

Die US 5,644,234 beschreibt ein Kernspinresonanz(MR)-Verfahren, wobei die Position einer Mikrospule in einem Objekt ermittelt werden soll.

Aus der WO 98/06342 sind ein Verfahren und eine Vorrichtung zur transcranialen magnetischen Stimulation des Gehirns bekannt, wobei ein etwa halbkugelförmiger von Spulen umwickelter Magnetkern zum Erzeugen eines Stimulationssignals verwendet wird. Mit der beschriebenen Vorrichtung und dem Verfahren soll die Sprachfunktion lokalisiert werden.

Verfahren und eine Vorrichtung für eine transcraniale magnetische Stimulation zur nicht-invasiven Lokalisation bestimmter Bereiche des Gehirns sind aus der EP 1 273 320 A1 der Anmelderin bekannt, deren Offenbarung bezüglich der Stimulation von Gehirnbereichen und insbesondere bezüglich der Erzeugung von Simulationsmodellen in diese Anmeldung aufgenommen wird.

Weiterhin sind Verfahren und Vorrichtungen zur nicht-invasiven Lokalisation und/oder Funktionsbeschreibung bestimmter Bereiche eines Gehirns, wie zum Beispiel primärer oder sekundärer Hirnareale zum Kartieren von Hirnfunktionen, aus den Europäischen Patentanmeldungen mit den Anmeldenummem 02 002 032.7 und 02 002 033.5 der Anmelderin bekannt.

Es ist eine Aufgabe der vorliegenden Erfindung eine Vorrichtung zum Berechnen einer Energiemenge zur Stimulation bestimmter Bereiche eines Gehirns vorzuschlagen, mit welchen die Genauigkeit der Stimulation eines bestimmten Bereiches verbessert werden kann. Weiterhin soll eine Vorrichtung zur einfacheren Lokalisation von Gehirnbereichen vorgeschlagen werden.

Diese Aufgabe wird durch eine Vorrichtung wie in den unabhängigen Ansprüchen definiert gelöst. Vorteilhafte Ausführungsformen ergeben sich aus den Unteransprüchen.

Unter Stimulation wird im Sinne der Erfindung nicht nur das aktive eigentliche Stimulieren eines bestimmten Gehirnbereiches oder -punktes verstanden, sondern auch das Anlegen oder Erzeugen von Signalen, welche eine Funktionsunterdrückung bestimmter Gehirnbereiche verursachen, häufig auch als funktionelle Läsion bezeichnet. Der Begriff "Stimulieren" soll demzufolge so verstanden werden, dass auch ein Blockieren oder Inhibieren von Hirnfunktionen durch bestimmte Stimulationssignale verursacht werden kann. Das Stimulieren oder Inhibieren eines Hirnbereiches erfolgt durch Anlegen bzw. Erzeugen von Signalen im Gehirn durch eine Stimulationsvorrichtung, wie zum Beispiel eine auf den Kopf aufgelegte von Strom durchflossene Spule, um im Gehirn durch Induktion elektrische Signale zu erzeugen, wobei die Signale eine bestimmte Impulsform oder eine Folge von Impulsen mit bestimmten Impulsformen und einer oder mehrerer vorgegebener Frequenzen sein können. Es hat sich gezeigt, dass bei Verwendung höherer Frequenzen zum Beispiel im Bereich von 50 Hz bestimmte Gehirnbereiche blockiert bzw. inhibiert werden können, so dass die von den betroffenen Gehirnbereichen zu erfüllenden Funktionen, wie zum Beispiel die Wahrnehmung eines externen Reizes, nicht mehr durchgeführt werden können. Demgegenüber erfolgt die Stimulation bevorzugt durch einen einzigen magnetischen bzw. dadurch induzierten elektrischen Impuls.

Bei einem Verfahren zum Stimulieren eines bestimmten Bereichs eines Gehirns mit einer Stimulationsvorrichtung, wie zum Beispiel einer Induktionsspule, wird die räumliche Struktur des Kopfes zum Beispiel mittels eines Kernspinresonanzverfahrens (MRI), einer Computertomografie (CT), einem Ultraschallverfahren oder einem anderen geeigneten Verfahren aufgenommen, um Daten zur Bestimmung zumindest eines Teils der anatomischen Struktur des Kopfes, wie zum Beispiel der Lage, Beschaffenheit und/oder Dicke von Knochen und der Lage und Art oder auch Dicke von Gewebe, wie zum Beispiel der Kopfhaut oder harten Hirnhaut zu erhalten.

Aus der so ermittelten räumlichen Struktur zumindest eines Teils des Kopfes werden bestimmte Eigenschaften der anatomischen Struktur(en) des Kopfes, wie zum Beispiel Schichten über dem Gehirn und bevorzugt auch die Lage und Struktur des Gehirns im Kopf ermittelt. Aus diesen Daten kann erfindungsgemäß bevorzugt automatisch berechnet werden, wie groß eine Energiemenge sein muss, um eine Stimulations- oder Induktionsvorrichtung anzusteuern, damit ein bestimmter Bereich des Gehirns möglichst präzise stimuliert werden kann. Somit kann erfindungsgemäß zum Beispiel ein von einer Induktionsvorrichtung erzeugtes Magnetfeld so eingestellt werden und bevorzugt dessen Feldstärke so festgelegt werden, dass ein gewünschter Bereich auf oder im Gehirn möglichst präzise mit einem einstellbaren dort induzierten Strom stimuliert werden kann, wobei zum Beispiel die dielektrischen Eigenschaften von über dem zu stimulierenden Bereich liegenden Gewebe- und Knochenstrukturen zur Einstellung des Magnetfeldes bzw. zur Festlegung der Energiemenge für die Induktionsvorrichtung berücksichtigt werden.

Da bisher die Einstellung der Energiemenge bzw. des Magnetfeldes manuell anhand von Schätzwerten durchgeführt wurde, ermöglicht das Verfahren das genaue Fokussieren einer bestimmten Energiemenge auf einen bestimmten definierten Bereich auf der Oberfläche des Gehirns oder in einer bestimmten Tiefe unterhalb der Oberfläche im Inneren des Gehirns, wodurch die genaue Stimulation eines definierten auch kleineren Bereichs möglich ist.

Mit dem Verfahren kann somit die transcraniale magnetische Stimulation eines bestimmten Bereichs des Gehirns verbessert und insbesondere präzisiert werden, wodurch zum Beispiel eine präzise nicht-invasive Lokalisation bestimmter Gehirnbereiche, wie zum Beispiel der oben erwähnten primären Hirnareale ermöglicht wird, so dass beispielsweise die präoperative chirurgische Planung verbessert werden kann. Weiterhin kann das Verfahren auch zur Untersuchung oder Heilung anderer Funktionsstörungen des Gehirns eingesetzt werden, wie zum Beispiel zum Diagnostizieren einer Epilepsie oder der Parkinsonschen Krankheit. Somit ermöglicht das erfindungsgemäße Verfahren die Durchführung einer Kartierung von Hirnfunktionen, ohne den Schädel einer Person öffnen zu müssen, um direkt auf das Gehirn zugreifen zu können.

Bevorzugt werden die Dicke und/oder die Art bzw. Beschaffenheit von Knochen und/oder Gewebe als Eigenschaften von anatomischen Schichten oder Strukturen im Kopf durch geeignete Aufnahmeverfahren, wie zum Beispiel Computertomografie zur Ermittlung der Knochenart und -Struktur oder Kernspinresonanzverfahren zur Ermittlung der Gewebearten oder -Struktur ermittelt. Verfahren zum Aufnehmen von Körperstrukturen und zum Zuordnen verschiedener Aufnahmen zueinander sind im Stand der Technik bekannt.

Vorteilhaft wird basierend auf den durch geeignete Verfahren ermittelten Eigenschaften von anatomischen Strukturen oder Schichten im Kopf eine Modellierung des Kopfes durchgeführt, wobei beispielsweise ein finites Mehr-Schalen-Model, bevorzugt ein 3-Schalen-Modell des Kopfes erstellt wird, so dass die Verteilung von elektrischen und/oder magnetischen Feldern im Kopf oder auf bzw. in bestimmten Bereichen des Gehirns zum Beispiel mit Finite-Elemente-Methoden berechnet werden können. Bezüglich der Erzeugung eines Simulationsmodells des Kopfes wird auf die EP 1 273 320 A1 verwiesen, deren diesbezügliche Offenbarung in diese Anmeldung aufgenommen wird. Ebenso kann ein Simulationsmodell der zur Stimulation verwendeten Induktionsvorrichtung erstellt werden, was ebenfalls in der EP 1 273 320. A1 beschrieben ist, deren diesbezügliche Lehre in diese Anmeldung aufgenommen wird. Anhand des Simulationsmodells des Kopfes und optional auch der Stimulationsvorrichtung kann ermittelt werden wie groß die Menge der der Stimulationsvorrichtung zuzuführenden Energie sein muss, wenn ein bestimmter Gehirnbereich stimuliert werden soll.

Bevorzugt werden an dem Kopf und/oder an der Induktionsvorrichtung Marker, besonders bevorzugt passive Marker angebracht, um ein Tracking der Induktionsvorrichtung und/oder des Kopfes vornehmen zu können. Die Referenzierung und das Tracking von Personen und/oder Instrumenten ist aus dem Stand der Technik bekannt und wird hier nicht näher beschrieben. Es wird ein Matching zwischen Induktionsvorrichtung und Kopf vorgenommen, um so die relative räumliche Lage zwischen Induktionsvorrichtung und Kopf möglichst präzise bestimmen zu können. Die Verwendung von Markern hat den Vorteil, dass eine untersuchte Person, nicht ortsfest positioniert werden muss, sondern dass auch bei einer sich frei im Raum bewegenden Person eine präzise Stimulation bestimmter Hirnbereiche vorgenommen werden kann.

Das Tracken der Induktionsvorrichtung oder TMS Spule zusammen mit dem Kopf ermöglicht das automatische Einstellen oder Korrigieren der Energiemenge, welche an die Induktionsvorrichtung abgegeben wird, um ein zur Stimulation eines Gehirnbereichs erforderliches Magnetfeld zu erzeugen, so dass zum Beispiel die Induktionsvorrichtung über den Kopf bewegt werden kann und die der Induktionsvorrichtung zugeführte Energie in Abhängigkeit von der relativen Lage zwischen Induktionsvorrichtung und Kopf so eingestellt wird, dass nur der gewünschte Bereich des Gehirns durch ein von der Induktionsvorrichtung erzeugtes Magnetfeld stimuliert wird. Befinden sich zum Beispiel Induktionsvorrichtung und Kopf in einem relativen Lageverhältnis, welches es nicht ermöglicht, dass der gewünschte Bereich des Gehirns stimuliert wird, so kann zum Beispiel die Energiezufuhr zur Induktionsvorrichtung unterbrochen werden.

Vorteilhaft wird die der Induktionsvorrichtung zuzuführenden Energiemenge auch in Abhängigkeit von dem zu stimulierenden bzw. zu inhibierenden Bereich des Gehirns eingestellt, so dass zum Beispiel in Abhängigkeit von der zumeist bekannten Funktion eines Gehirnbereichs, wie zum Beispiel visueller, auditorischer oder motorischer Kortex, die der Induktionsvorrichtung zuzuführende Energiemenge eingestellt werden kann. Insbesondere ist es vorteilhaft die Tiefe eines zu stimulierenden Gehirnbereichs unterhalb der Oberfläche des Gehirnes oder des Kopfes zu ermitteln, um die der Induktionsvorrichtung zuzuführende Energiemenge optimal einzustellen.

Die Stimulations- oder Induktionsvorrichtung weist bevorzugt mindestens eine Spule, zum Beispiel in Form einer 8 auf, an welche elektrische Impulse angelegt werden können.

Die Stimulation eines Gehirnareals erfolgt bevorzugt durch an die Stimulations- oder Induktionsvorrichtung angelegte elektrische Impulse, welche eine Anstiegszeit im Bereich von einer Mikrosekunde bis einer Millisekunde und eine Dauer von 10 bis 1000 Mikrosekunden haben können. Dabei können die einzelnen Impulse mit einem periodischen Muster angelegt werden.

Bevorzugt erfolgt eine optische Anzeige der durch eine Aufnahme ermittelten räumlichen Struktur der Gehirnoberfläche, vorteilhaft zusammen mit einer Anzeige des simulierten Stimulationsbereiches bei der momentanen Lage der Induktionsvorrichtung. Unter Zuhilfenahme einer solchen optischen Anzeige kann eine Bedienperson zum Beispiel durch seitliches Kippen der Induktionsvorrichtung und/oder Bewegen der Induktionsvorrichtung auf den Kopf zu oder von dem Kopf weg die Position und Größe des Stimulationsbereiches verändern, wobei zum Beispiel ein möglichst kleiner Fokussierpunkt angestrebt werden kann, um zum Beispiel eine möglichst hohe räumliche Auflösung bei der Lokalisation bestimmter Gehirnbereiche zu erhalten.

Vorteilhaft erfolgt die Positionierung der Induktionsvorrichtung relativ zu dem Kopf automatisch, wobei die Induktionsvorrichtung durch beispielsweise einen mit mehreren Freiheitsgraden bewegbaren Roboterarm so positioniert wird, dass eine Vielzahl von Punkten auf der Oberfläche des Gehirns mit einem möglichst kleinen Fokussierpunkt stimuliert werden können. Hierzu kann der bewegliche Roboterarm fest an dem Kopf befestigt werden. Dabei können zum Beispiel von einer Mustererkennungssoftware geeignete Stimulationspunkte auf der Hirnoberfläche ausgewählt werden und/oder Stimulationspunkte von einer Bedienperson vorgegeben werden, welche dann von dem Roboterarm automatisch so angefahren werden, dass die positionierte Induktionsvorrichtung einen möglichst kleinen Bereich der Hirnoberfläche stimuliert. Zur automatischen Positionierung kann ein Simulationsmodell der Induktionsvorrichtung und/oder des Kopfes verwendet werden.

Es ist vorteilhaft die zur Stimulation verwendete Energie zum Beispiel automatisch in Abhängigkeit von der relativen Position zwischen Kopf und Stimulationsvorrichtung einzustellen.

Bevorzugt wird die Energiemenge zum Ansteuern der Induktionsvorrichtung zur Stimulation des bestimmten Bereichs des Gehirns in Abhängigkeit von der gewünschten zu erzielenden Funktion eingestellt, also in Abhängigkeit davon, ob eine Anregung oder eine Unterdrückung bzw. Inhibition eines bestimmten Gehirnbereichs erreicht werden soll. Dabei können auch zusätzlich geeignete Signal- oder Impulsformen gewählt werden, welche ein Stimulieren bzw. Inhibieren eines bestimmten Gehirnbereichs ermöglichen.

Bei einem Verfahren zum Zuordnen einer Stimulationsreaktion zu einem Gehirnbereich wird der Gehirnbereich mit mindestens einer oder einer Mehrzahl von Stimulationsvorrichtungen stimuliert, welche zum Beispiel nebeneinander in einer Ebene oder auf einer Elipsoid- oder Kugelfläche angeordnet sein können und wobei eine Stimulationsreaktion durch mindestens einen Sensor gemessen wird. Ein solcher Sensor kann beispielsweise ein Sensor zur Messung einer Muskelerregung oder Zuckung sein, welcher an einem bestimmten Bereich eines Körpers angebracht wird. Beispielsweise können ein oder mehrere Sensoren an einer öder mehreren Fingerkuppen, einem Arm, einem Bein und/oder einem Fuß angebracht werden, um zu ermitteln, ob die Stimulation eines oder mehrerer Gehirnbereiche eine oder mehrere Reaktionen an den betreffenden Körperstellen, wie zum Beispiel eine Muskelzuckung, verursachen. Durch solche Sensoren kann nicht nur gemessen werden, ob überhaupt eine Reaktion vorliegt, sondern es kann gemäß einer Ausführungsform auch die Intensität der Stimulationsreaktion oder Muskelzuckung gemessen werden, wodurch es möglich ist zu ermitteln, ob zum Beispiel ein oder mehrere gleichzeitig oder nacheinander stimulierte Gehimbereiche eine eher starke oder eine eher schwache Stimulationsreaktion hervorrufen. Vorteilhaft werden eine oder auch mehrere gemessene Stimulationsreaktionen dem oder den stimulierten Gehirnbereich (en) zugeordnet, wodurch einfach ermittelt und dargestellt werden kann, ob zum Beispiel ein gerade stimulierter Gehirnbereich eine Reaktion an einem bestimmten Körperbereich hervorruft oder nicht. Ergänzend kann auch die Intensität der durch die Stimulation hervorgerufenen Stimulationsreaktion dargestellt werden. Hierzu kann zum Beispiel einer bestimmten Intensität einer gemessenen Stimulationsreaktion eine bestimmte Farbe zugeordnet werden, so dass beispielsweise schwache Stimulationsreaktionen durch eine Blaueinfärbung des gerade stimulierten Gehirnbereichs in einer Simulationsdarstellung des Gehirns gekennzeichnet werden, mittelstarke Stimulationsreaktionen können zum Beispiel orange gekennzeichnet werden und starke Stimulationsreaktionen können zum Beispiel durch eine Roteinfärbung der Gehirndarstellung zum Beispiel auf einem Bildschirm gekennzeichnet werden.

Das gerade beschriebene Verfahren zum Zuordnen mindestens einer Stimulationsreaktion zu mindestens einem Gehirnbereich kann in Verbindung mit, oder auch unabhängig von, einzelnen der oben beschriebenen Verfahrensschritte zum Stimulieren eines bestimmten Bereiches eines Gehirns durchgeführt werden.

Nach einem Aspekt bezieht sich die Erfindung auf eine Vorrichtung zum Stimulieren eines oder mehrerer Bereiche des Gehirns mit einer Stimulations- oder Induktionsvorrichtung, wobei eine Aufnahmevorrichtung, wie beispielsweise ein Kernspintomograph, eine Ultraschall- oder eine Computertomografievorrichtung vorgesehen ist, um die räumliche anatomische Struktur des Kopfes mit den einzelnen anatomischen Schichten oder Strukturen und/oder die räumliche Struktur des Gehirns zu erfassen. Weiterhin ist erfindungsgemäß eine Recheneinheit vorgesehen, mit welcher magnetische und/oder elektrische Eigenschaften der anatomischen Strukturen des Kopfes und/oder des Gehirns ermittelt werden können und eine an eine Stimulations- oder Induktionsvorrichtung abzugebende Energiemenge zur Stimulation eines bestimmten vorgegebenen Bereichs des Gehirns berechnet und basierend auf den so ermittelten Eigenschaften eingestellt werden kann.

Bevorzugt weist die erfindungsgemäße Vorrichtung eine Anzeigevorrichtung zur Anzeige des durch die Induktionsvorrichtung stimulierbaren oder stimulierten Bereichs auf oder in dem Gehirn auf, so dass eine Bedienperson der Vorrichtung anhand der Anzeige erkennen kann, ob bei der momentanen Position der Induktionsvorrichtung der gewünschte Bereich stimuliert bzw. inhibiert werden kann oder ob die Position der Induktionsvorrichtung verändert werden muss.

Mit der Erfindung kann eine Vorrichtung zum Zuordnen einer Stimulationsreaktion zu einem Gehirnbereich geschaffen werden mit mindestens einer Stimulationsvorrichtung zum Stimulieren des Gehirnbereichs, mindestens einem Sensor zum Messen einer Stimulationsreaktion und mindestens einer Recheneinheit, durch welche die mindestens eine gemessene Stimulationsreaktion dem mindestens einem stimulierten Gehirnbereich zugeordnet werden kann. Eine solche Vorrichtung ermöglicht demzufolge das unmittelbare und automatische Zuordnen einer durch zum Beispiel einen oder mehrere Sensoren gemessenen Stimulationsreaktion zu zum Beispiel einem oder mehreren stimulierten Gehirnbereichen, wodurch es beispielsweise möglich ist einer Bedienperson auf einem Bildschirm, welcher ein Simulationsmodell des Gehirns zeigt, durch geeignete Einfärbung des gerade stimulierten Gehirnbereichs anzuzeigen ob und wie stark eine Reaktion auf die gerade durchgeführte Stimulation ist. Somit ist es möglich, dass zum Beispiel eine Stimulationsvorrichtung über einen Kopf hinweggeführt wird und falls Stimulationsreaktionen gemessen werden, können diese als Einfärbungen eines auf einem Bildschirm dargestellten Gehirnmodells gekennzeichnet werden, so dass bei geeigneter Bewegung der Stimulationsvorrichtung über den Kopf nach und nach zum Beispiel größere Bereiche des Gehirns eingefärbt werden können, um zum Beispiel ein sogenanntes Brain Mapping durchzuführen. Somit kann ermittelt werden, welcher Bereich des Gehirns einen stärkeren oder schwächeren Einfluss auf einen bestimmten Körperteil hat, an welchem zum Beispiel ein oder mehrere der oben beschriebenen Reaktionssensoren angebracht sind. Dabei wird bevorzugt die von dem Navigationssystem und/oder einer Simulation des Gehirns ermittelte Position des stimulierten Gehirnbereichs verwendet, um dieser Position die gemessene Intensität der Stimulationsreaktion zuzuordnen und zum Beispiel durch verschiedene Farbeinfärbungen zu kennzeichnen.

Des weiteren bezieht sich die Erfindung auf ein System mit einer wie oben beschriebenen Vorrichtung und einer Stimulations- oder Induktionsvorrichtung, welche zum Beispiel eine einfache Spule oder eine Spule in Form einer 8 ist.

Bevorzugt sind auf bekannte Art Marker an der Induktionsvorrichtung und/oder einem Kopf befestigt, so dass das Lageverhältnis zwischen der Induktionsvorrichtung und dem Objekt oder Kopf auf bekannte Art zum Beispiel durch Kameras erfasst werden kann, wodurch die Induktionsvorrichtung zu einer für die Stimulation eines bestimmten Bereichs des Gehirns vorteilhaften Position relativ zum Objekt oder Kopf navigiert werden kann.

Die Erfindung wird nachfolgend anhand bevorzugter Ausführungsformen beschrieben werden. Es zeigen:
- Figur 1: ein erfindungsgemäßes System zum automatischen Einstellen der einer Induktionsvorrichtung zuzuführenden Energiemenge;
- Figur 2: eine schematische Darstellung eines Simulationsmodell des Kopfes mit drei Schalen; und
- Figur 3: ein Ablaufdiagramm einer Ausführungsform des Verfahrens.

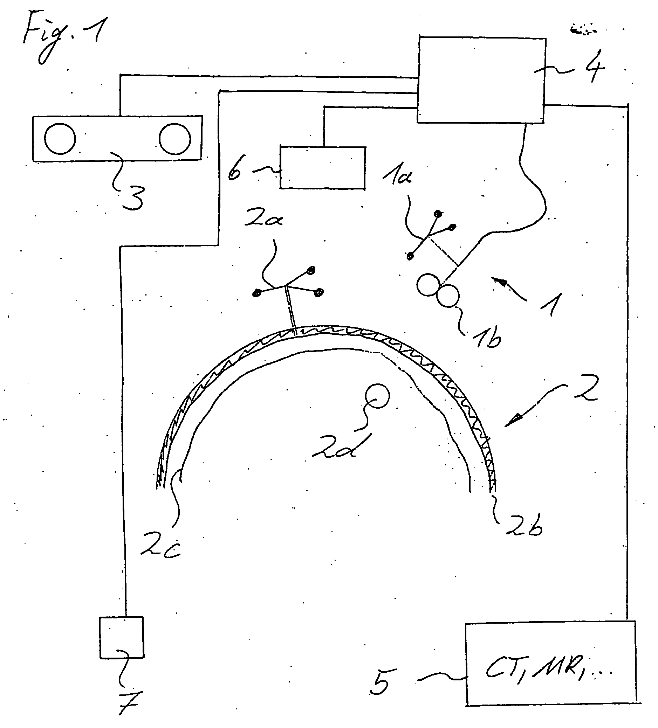

Figur 1 zeigt einen Kopf 2, dessen anatomische Struktur durch eine Kernspinresonanz- und/oder Computertomografievorrichtung 5 ermittelt wurde, so dass der Verlauf und die Dicke des Schädelknochens, die Qualität des Schädelknochens, wie zum Beispiel der Grad der Kalkablagerung, die Struktur, Dicke und die Art des Gewebes, wie zum Beispiel der Kopfhaut und der harten Hirnhaut, sowie die Lage des Gehirns 2c im Kopf 2 unterhalb der vorher genannten anatomischen Strukturen bekannt ist. Auf oder in dem Gehirn 2c befindet sich ein durch magnetische Felder von einer Induktionsvorrichtung 1 zu stimulierender Bereich 2d. Am Kopf 2 sind auf bekannte Art Marker 2a angebracht, welche von einem Kamerasystem 3 erfasst werden können, so dass die räumliche Lage des Kopfes 2 in einer mit dem Kamerasystem 3 verbundenen Recheneinheit 4 ermittelt und auf einer Anzeige 6 dargestellt werden kann.

Mit der Recheneinheit 4 ist die Stimulations- oder Induktionsvorrichtung 1 verbunden, an welcher ebenfalls Marker 1a angebracht sind, die von dem Kamerasystem 3 erfasst werden können. Somit kann die relative Lage zwischen der Stimulationsvorrichtung 1 und dem Kopf 2 ermittelt werden. Die Stimulationsvorrichtung 1 weist eine in Form einer 8 geformte Spule 1b zur Erzeugung eines Magnetfeldes auf, mit welchem der Bereich 2d auf dem Gehirn 2c stimuliert werden soll.

Hierzu wird anhand der vorher ermittelten anatomischen Strukturen oder draus berechneten magnetischen und/oder elektrischen Eigenschaften des Kopfes 2 und bevorzugt unter Verwendung eines Simulationsmodells des Kopfes 2 und/oder eines Simulationsmodells der Stimulationsvorrichtung 1 und/oder einem Simulationsmodell des von der Stimulationsvorrichtung 1 erzeugten elektrischen Feldes die Energiemenge ermittelt, welche zum Beispiel von der Recheneinheit 4 eingestellt und an die Spule 1b der Stimulationsvorrichtung 1 übertragen wird, um eine möglichst optimale Stimulation des Bereichs 2b durchführen zu können.

Dabei ist die Berechnung der der Spule 1b zuzuführenden Energiemenge bevorzugt so, dass in Abhängigkeit vom relativen Lageverhältnis von Stimulationsvorrichtung 1 bzw. Spule 1b und Kopf 2 die der Spule 1b zuzuführende Energie automatisch verändert wird, wodurch beispielsweise die Spule 1b über den Kopf 2 hinweg bewegt werden kann und nur bei Positionen, bei welchen der Bereich 2b des Gehirns 2c stimuliert wird, auch Energie der Spule 1b zugeführt wird.

Optional kann ein Sensor 7 zum Beispiel an einem Arm einer untersuchten Person angeordnet werden, um zum Beispiel die Zuckung eines bestimmten Muskels zu messen. Wird der Bereich 2b des Gehirns 2c durch die Induktionsvorrichtung 1 stimuliert und wird nach dieser Stimulation durch den Sensor 7 eine Reaktion zum Beispiel in Form einer Muskelzuckung gemessen, so kann die Intensität der gemessenen Reaktion an die Recheneinheit 4 weitergegeben werden, welche die durch den Sensor 7 gemessene Reaktion dem stimulierten Bereich 2d zuordnet. Die Intensität der Reaktion kann auf der Anzeige 6 anhand eines Simulationsmodells des Gehirns 2c dargestellt werden, wobei der stimulierte Bereich 2d in Abhängigkeit von der durch den Sensor 7 gemessenen Intensität der Stimulationsreaktion in einem bestimmten Farbton eingefärbt wird, welcher zum Beispiel eindeutig vorgegeben und einer Intensität zugeordnet ist.

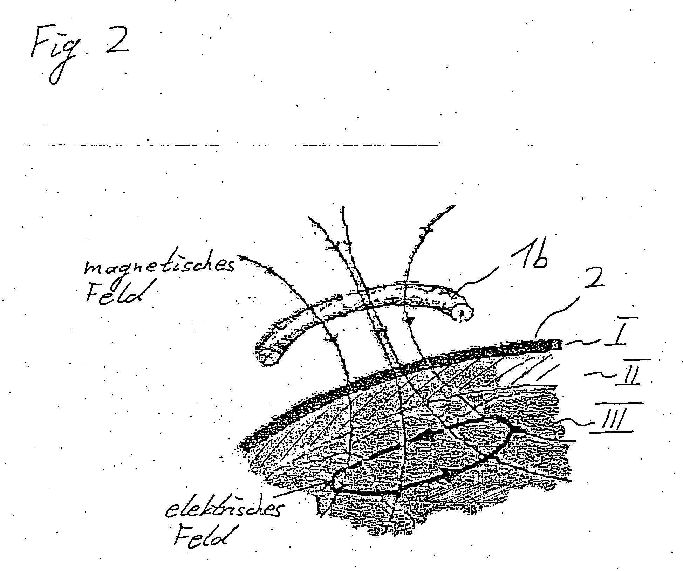

Figur 2 zeigt schematisch den Verlauf von magnetischen und elektrischen Feldlinien, welche von einer über einem Kopf 2 liegenden Spule 1b erzeugt werden. Sind die anatomischen Strukturen des Kopfes 2 zum Beispiel aufgrund von vorherigen Aufnahmen bekannt, so kann ein Simulationsmodell des Kopfes 2 mit drei Schalen zum Beispiel zur Modellierung der Kopfhaut, der Knochenstruktur und des Gehirns erstellt werden, welche schematisch in Figur 2 gezeigt sind.

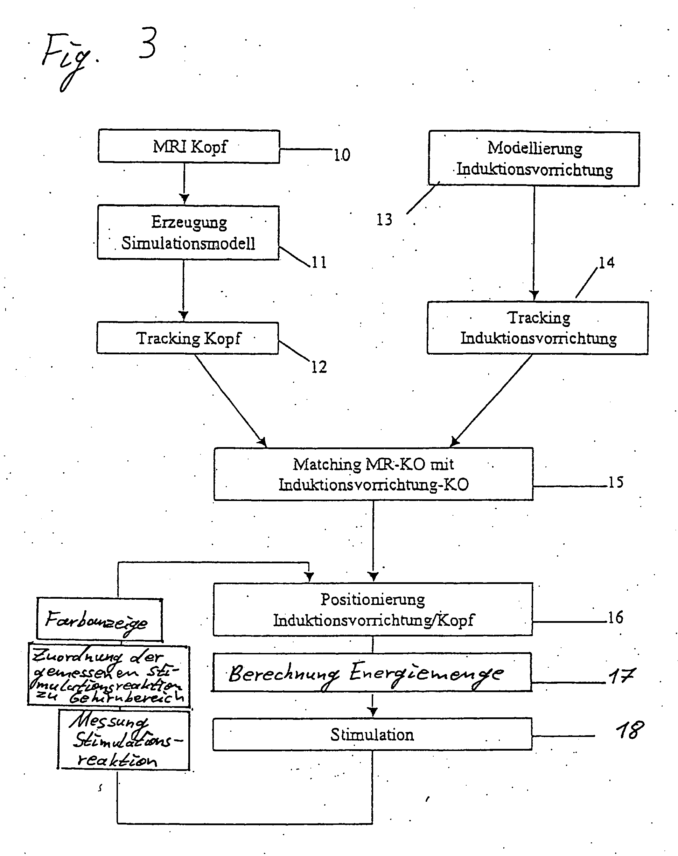

Figur 3 zeigt schematisch das Ablaufdiagramm einer Ausführungsform des Verfahrens. In einem ersten Schritt 10 wird durch Kernspinresonanz (MRI) und Computertomografie (CT) die räumliche Struktur eines Kopfes mit Gehirn aufgenommen. Die in Schritt 10 gewonnen Daten werden in Schritt 11 verwendet, um ein Simulationsmodell des aufgenommenen Kopfes zu erzeugen, wobei zum Beispiel wie oben beschrieben die Kopfhaut, der Schädelknochen und das Gehirn als drei Bereiche I, II und III modelliert werden, welche jeweils eine charakteristische Dielektrizitätskonstante und einen charakteristischen Leitwert aufweisen. Der Kopf ist mit einem Referenzstern 2a verbunden, wie in Fig. 1 gezeigt, wodurch dessen räumliche Position jederzeit einfach erfasst werden kann (Tracking in Schritt 12).

In Schritt 13 wird die verwendete Induktionsvorrichtung 1 modelliert, wobei zur Modellierung Daten verwendet werden können, welche aus einer genauen Untersuchung der räumlichen Struktur von in der Induktionsvorrichtung enthaltenen Leitern bzw. Spulen gewonnen wurden, so dass ein durch die Induktionsvorrichtung erzeugbares magnetisches Feld relativ genau berechnet und simuliert werden kann. Weiterhin kann die Induktionsvorrichtung auch durch Auswertung von Messungen in dem durch die Induktionsvorrichtung erzeugten magnetischen Feld modelliert werden. Durch die Modellierung kann ein Fokussierbereich einer konkret verwendeten Induktionsvorrichtung relativ genau bestimmt werden. Die Induktionsvorrichtung wird, wie in Fig. 1 gezeigt, mit einem Referenzstern 1a verbunden, wodurch die Induktionsvorrichtung 1 ebenso wie der mit einem Referenzstern 2a verbundene Kopf 2 getrackt werden kann.

Es wird in Schritt 15 ein Abgleich (Matching) der Koordinaten des Kopfes und damit der räumlichen Lage der durch MRI und CT gewonnenen Struktur des Gehirns mit den Koordinaten der Induktionsvorrichtung vorgenommen, wodurch die relative räumliche Lage der Induktionsvorrichtung zu der durch die Kernspintomographie ermittelten räumlichen Struktur des Kopfes, insbesondere des Gehirns, erhalten werden kann. Unter Verwendung dieser nun bekannten räumlichen Lagebeziehung kann in Schritt 16 eine Positionierung der Induktionsvorrichtung am Kopf erfolgen, wobei die Modellierungsdaten der Induktionsvorrichtung und die Modellierungsdaten des Kopfes verwendet werden, um den bei einem Stromfluss durch die Induktionsvorrichtung erzeugten Induktionsbereich auf dem Gehirn zu simulieren.

Ist die Positionierung so durchgeführt worden, dass in der Simulation zum Beispiel ein möglichst kleiner vorgegebener Bereich des Gehirns durch die Induktionsvorrichtung stimuliert wird, so wird in Schritt 17 die der Induktionsvorrichtung zuzuführende Energiemenge berechnet, um den vorgegebenen Bereich mit der gewünschten Intensität stimulieren zu können. Wird diese Energiemenge als Strom der Spule der Induktionsvorrichtung zugeführt, so wird die Stimulation dieses durch eine Simulation vorab ermittelten Bereiches in Schritt 18 durchgeführt.

Optional ist es möglich nach der Stimulation in Schritt 18 eine Stimulationsreaktion zu messen und die gemessene Stimulationsreaktion automatisch dem gerade stimulierten Gehirnbereich zuzuordnen und die Intensität der gemessenen Reaktion zum Beispiel als farbige Einfärbung auf einer Darstellung eines Simulationsmodells des Gehirns anzuzeigen.

Ein Beobachter kann dann zum Beispiel feststellen, was eine Person für spezifische Reaktionen zeigt, wenn dieser bestimmte Bereich stimuliert wurde, wobei anhand dieser Reaktionen, wie zum Beispiel dem Zucken eines Muskels, Störung des Sprachverhaltens oder ähnliches ermittelt werden kann, ob der stimulierte Bereich des Gehirns eine bestimmte Funktionalität besitzt. Wird dieser Vorgang der Positionierung, Berechnung der Energiemenge und Stimulation bei einer Vielzahl von Bereichen des Gehirns durch Wiederholen der Schritte 16 bis 18 durchgeführt, so kann eine Kartierung von Hirnfunktionen vorgenommen werden, wobei insbesondere die primären Hirnareale der untersuchten Person lokalisiert werden können. Dabei muss das gewünschte induzierte elektrische Feld unter Verwendung der Simulationsmodelle wieder neu berechnet werden, wobei zum Beispiel bezüglich der Induktionsvorrichtung bis zu sieben Freiheitsgrade (drei für Translation, drei für Rotation und einer für Spulenstrom) zu berücksichtigen sind.

## Patentansprüche

1. Vorrichtung zum Berechnen einer Energiemenge zur Stimulation eines bestimmten Bereichs eines Gehirns mit einer Vorrichtung (5) zur Aufnahme der räumlichen anatomischen Strukturen eines Kopfes (2), einer Recheneinheit (4) zum Ermitteln der elektrischen und/oder magnetischen Eigenschaften der anatomischen Strukturen des Kopfes (2) und einer Recheneinheit (4) zum Berechnen der Energiemenge zum Ansteuern einer Stimulationsvorrichtung (1) zum Stimulieren des bestimmten Bereichs (2b) des Gehirns (2c) mit einem einstellbaren dort induzierten Strom.

2. Vorrichtung nach dem vorhergehenden Anspruch mit einer Anzeigevorrichtung (6), um einen zu stimulierenden oder stimulierten Bereich (2d) des Gehirns (2c) anzuzeigen.

3. System mit einer Vorrichtung nach einem der beiden vorhergehenden Ansprüche und einer Stimulationsvorrichtung (1).

4. System nach dem vorhergehenden Anspruch, wobei an der Stimulationsvorrichtung (1) und/oder an dem Objekt (2) Marker (1a, 2a) vorgesehen sind und mit einem Navigationssystem (3, 4) zum Ermitteln der relativen räumlichen Lage zwischen Stimulationsvorrichtung (1) und Objekt (2).

## Claims

1. A device for calculating an energy amount for stimulating a particular area of a brain, comprising: a device (5) for recording the spatial anatomical structures of a head (2); a computational unit (4) for determining the electrical and/or magnetic properties of the anatomical structures of the head (2); and a computational unit (4) for calculating the energy amount for guiding a stimulation device (1) for stimulating the particular area (2b) of the brain (2c) using a settable current induced therein.

2. The device according to the preceding claim, comprising a display device (6) for displaying a stimulated area (2d) of the brain (2c) or area (2d) of the brain (2c) to be stimulated.

3. A system comprising a device according to any one of the preceding two claims and a stimulation device (1).

4. The system according to the preceding claim, wherein markers (1a, 2a) are provided on the stimulation device (1) and/or on the object (2), and comprising a navigation system (3, 4) for determining the relative spatial position of the stimulation device (1) and the object (2).

## Revendications

1. Dispositif pour calculer une quantité d'énergie pour la stimulation d'une zone définie d'un cerveau avec un dispositif (5) pour l'enregistrement des structures anatomiques spatiales d'une tête (2), une unité de calcul (4) pour déterminer les propriétés électriques et/ou magnétiques des structures anatomiques de la tête (2) et une unité de calcul (4) pour calculer la quantité d'énergie pour déclencher un dispositif de stimulation (1) pour la stimulation de la zone (2b) définie du cerveau (2c) avec un courant induit à cet endroit et réglable.

2. Dispositif selon la revendication précédente comprenant un dispositif d'affichage (6) pour afficher une zone (2d) à stimuler ou stimulée du cerveau (2c).

3. Système comprenant un dispositif selon l'une quelconque des deux revendications précédentes et un dispositif de stimulation (1).

4. Système selon la revendication précédente, dans lequel des marqueurs (1a, 2a) sont prévus sur le dispositif de stimulation (1) et/ou sur l'objet (2) et avec un système de navigation (3, 4) pour déterminer la position spatiale relative entre le dispositif de stimulation (1) et l'objet (2).
